(19)

Europäisches
Patentamt
European
Patent Office
Office européen
des brevets

(11) **EP 3 488 877 A1**

(12) **EUROPEAN PATENT APPLICATION**
published in accordance with Art. 153(4) EPC

(43) Date of publication:
**29.05.2019 Bulletin 2019/22**

(21) Application number: **17834335.6**

(22) Date of filing: **25.07.2017**

(51) Int Cl.:
*A61L 27/18* (2006.01)     *A61K 35/28* (2015.01)
*A61K 35/32* (2015.01)     *A61K 35/33* (2015.01)
*A61L 27/06* (2006.01)     *A61L 27/12* (2006.01)
*A61L 27/22* (2006.01)     *A61L 27/24* (2006.01)
*A61L 27/38* (2006.01)     *A61L 27/44* (2006.01)
*A61L 27/56* (2006.01)

(86) International application number:
**PCT/JP2017/026905**

(87) International publication number:
**WO 2018/021333 (01.02.2018 Gazette 2018/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **25.07.2016   JP 2016145829**

(71) Applicants:
• **UBE Industries, Ltd.**
  **Ube-shi, Yamaguchi 755-8633 (JP)**
• **Kurume University**
  **Kurume-shi**
  **Fukuoka 830-0011 (JP)**

(72) Inventors:
• **HAGIHARA, Masahiko**
  **Ube-shi**
  **Yamaguchi 755-8633 (JP)**
• **OHTA, Keisuke**
  **Kurume-shi**
  **Fukuoka 831-0011 (JP)**
• **HIRASHIMA, Shingo**
  **Kurume-shi**
  **Fukuoka 830-0011 (JP)**

(74) Representative: **Plougmann Vingtoft a/s**
  **Strandvejen 70**
  **2900 Hellerup (DK)**

(54) **IMPLANT AND KIT FOR TREATMENT OF BONE LESION SITE, AS WELL AS METHOD FOR TREATING BONE LESION SITE**

(57)     The present invention pertains to an implant for treatment of a bone lesion site, the implant including a polymer porous film and a layer containing a biocompatible material, wherein the polymer porous film is a polymer porous membrane of a three-layer structure having a porous surface layer A, a porous surface layer B, and a macrovoid layer interposed between the surface layer A and the surface layer B; the average pore diameter of the pores in surface layer A is smaller than the average pore diameter of the pores in surface layer B; the macrovoid layer has a partition joined to the surface layers A and B and a plurality of macrovoids enclosed by the partition and the surface layers A and B; and the pores in the surface layers A and B communicate with the macrovoids.

FIG. 1

**Description**

FIELD

The present invention relates to an implant and a kit for treating a bone injury site, and a method for treating the bone injury site.

BACKGROUND

Bone injury and healing

Numerous techniques using instruments or measures have long been attempted for healing bone injuries including fractures and bone loss by methods of fixing affected parts with plates or screws, for example, from the viewpoint of patients' QOL. In recent years, bone disease healing materials or instruments having various structural, functional and·biocompatible advantages have been created and utilized for medical care (NPL 1, PTL 1). For example, titanium mesh having properties of high robustness, flexibility and life adaptability is used for treatment of many bone injuries and intraoral diseases, for example. Titanium mesh can provide a stable wound healing space, however, the mesh does not have its own effect of healing bone injuries.

Furthermore, methods for accelerating the healing of bone injuries including fractures and bone loss have been attempted also from the viewpoint of regenerative medicine. For example, a method that involves culturing cells such as mesenchymal stem cells in biomaterials or bioabsorbable materials such as collagen or apatite, to prepare a complex and using the complex as a complement to an affected part has been reported (PTL 2 and PTL 3). Moreover, there are many discussions on whether or not coexistence with stem cells can contribute to therapeutic efficacy (NPL 2). These methods lead to cases where the methods are not suitable for large injuries·losses or complicated shapes or time-consuming cases. Furthermore, it has not been reported that the above biomaterials or bioabsorbable materials themselves accelerate the healing of bone injuries.

There is demand for a convenient and effective method of treating bone injury that is suitable for a variety of wound surfaces including sites with extensive loss and sites with complex shapes.

Porous polyimide film

Porous polyimide films have been utilized in the prior art for filters and low permittivity films, and especially for battery-related purposes, such as fuel cell electrolyte membranes and the like. PTLs 4 to 6 describe porous polyimide films with numerous macro-voids, having excellent permeability for gases and the like, high porosity, excellent smoothness on both surfaces, relatively high strength and, despite high porosity, also excellent resistance against compression stress in the film thickness direction. All of these are porous polyimide films formed via amic acid.

A cell culturing method comprising steps of applying cells to a porous polyimide film and culturing them has been reported (PTL 7).

PRIOR ART DOCUMENTS

PATENT LITERATURE

PTL 1: Japanese Patent No. 3029266
PTL 2: Japanese Patent No. 4412537
PTL 3: Japanese Patent No. 4950269
PTL 4: WO2010/038873
PTL 5: JP2011-219585A
PTL 6: JP2011-219586A
PTL 7: WO2015/012415

NON PATENT LITERATURE

NPL 1: F.G. Lyons et al. / Biomaterials 31 (2010) 9232-9243

NPL 2: S. Wu et al. / Materials Science and Engineering R 80 (2014)1-36

DISCLOSURE OF THE INVENTION

PROBLEMS TO BE SOLVED BY THE INVENTION

It is an object of the invention to provide convenient and effective means for treating bone injury, that is applicable for many different wound surfaces.

MEANS FOR SOLVING THE PROBLEMS

The present inventors have discovered that a porous polymer film with a three dimensional structure resembling a bone marrow structure can accelerate the healing of bone injury sites. The present inventors have also discovered that the cells exhibit excellent proliferative capacity on the porous polymer film.
Furthermore, the present inventors have discovered that through combination of a biopolymer such as collagen to be generally used for culturing mesenchymal stem cells and osteoblasts with the above porous polymer film, the proliferative capacity of these cells can be further improved, and thus have completed the present invention.
Namely, the present invention includes the following aspects.

[1] An implant for treating a bone injury site, the implant comprising:

a porous polymer film; and
a layer containing a biocompatible material;

wherein the porous polymer film is a three-layer structure porous polymer film having a surface layer A and a surface layer B, the surface layers having a plurality of pores, and a macrovoid layer sandwiched between the surface layers A and B;
wherein an average pore diameter of the pores present in the surface layer A is smaller than an average pore diameter of the pores present in the surface layer B;
wherein the macrovoid layer has a partition wall bonded to the surface layers A and B, and a plurality of macrovoids surrounded by the partition wall and the surface layers A and B; and
wherein the pores in the surface layers A and B communicate with the macrovoids.
[2] The implant according to [1], wherein the layer containing the biocompatible material is arranged on the surface layer A of the porous polymer film.
[3] The implant according to [1] or [2], wherein the biocompatible material is a compound selected from the group consisting of collagen, silicone, fibronectin, laminin, polylysine, polylactide, polyglycolide, polycaprolactone, polyhydroxybutyrate, polylactide-co-caprolactone, polycarbonate, biodegradable polyurethane, polyetherester, polyesteramide, hydroxyapatite, a complex of collagen and $\beta$-TCP, and a combination thereof.
[4] The implant according to any one of [1] to [3], wherein the biocompatible material is collagen.
[5] The implant according to any one of [1] to [4], wherein an average pore diameter of the surface layer A is 0.01 to 50 $\mu$m.
[6] The implant according to any one of [1] to [5], wherein an average pore diameter of the surface layer B is 20 to 100 $\mu$m.
[7] The implant according to any one of [1] to [6], wherein a film thickness of the porous polymer film is 5 to 500 $\mu$m.
[8] The implant according to any one of [1] to [7], wherein the porous polymer film is a porous polyimide film.
[9] The implant according to [8], wherein the porous polyimide film is a porous polyimide film comprising a polyimide derived from tetracarboxylic dianhydride and diamine.
[10] The implant according to [8] or [9], wherein the porous polyimide film is a colored porous polyimide film that is obtained by molding a polyamic acid solution composition comprising a polyamic acid solution derived from tetracarboxylic dianhydride and diamine, and a colored precursor, and subsequently heat-treating the resultant composition at 250°C or higher.
[11] The implant according to any one of [1] to [7], wherein the porous polymer film is a porous polyethersulfone film.
[12] The implant according to any one of [1] to [11], on which cells have been supported.
[13] The implant according to [12], wherein the cells are bone marrow cells, mesenchymal stem cells, fibroblasts, osteoblasts, or cells derived therefrom.
[14] The implant according to any one of [1] to [13], further comprising a metal mesh, wherein the layer containing the biocompatible material, the porous polymer film and the metal mesh are arranged in this order.
[15] The implant according to [14], wherein the metal mesh comprises titanium, stainless steel, titanium-coated steel, titanium nitride or a combination thereof.

[16] A kit for treating a bone injury, the kit comprising:

the implant according to any one of [1] to [15];
a material required for a bone injury treatment surgery; and
an instruction manual.

[17] A method for treating a bone injury site, the method comprising applying the implant according to any one of [1] to [15] to the bone injury site.

EFFECTS OF THE INVENTION

The present invention makes it possible to conveniently and effectively treat bone injuries including significant loss sites and complicated shape sites. A porous polymer film to be used in the present invention is flexible, and enables to retain and grow cells in its unique three dimensional structure. Cells of interest are supported on the porous polymer film and then the film is applied to a bone injury site, making it possible to accelerate the treatment of the bone injury site. Moreover, a biopolymer such as collagen to be used for culturing mesenchymal stem cells and osteoblasts is combined with the porous polymer film, so that the proliferative capacity of these cells can further be improved and treatment of the bone injury site can also be accelerated. Furthermore, cells are concentrated in the porous polymer film, and thus the number of cells in the vicinity of the wound surface is increased and osteogenesis can be accelerated.

BRIEF DESCRIPTION OF DRAWINGS

Fig. 1 shows a set of scanning electron micrographs of a porous polyimide film having a bone marrow-like structure. A scanning electron micrograph shows a cross-section, and the A-surface (mesh structure surface) and B-surface (large hole structure surface) of the porous polyimide film. For comparison, a scanning electron micrograph of bone marrow is also shown (panel (b)).

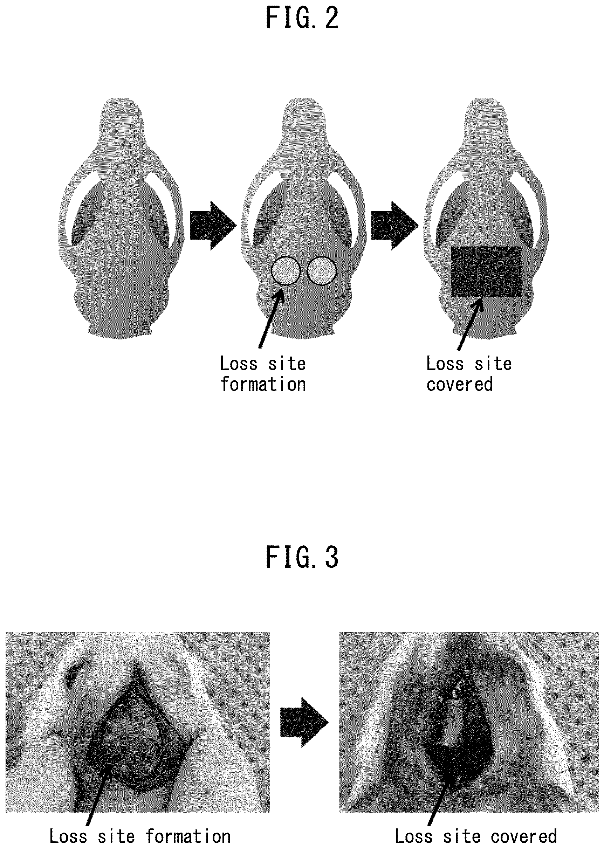

Fig. 2 shows a rat cranial bone loss model.
Fig. 3 shows photographs of a technique for covering a site of loss with a porous polyimide film sheet, in a rat cranial bone loss model.
Fig. 4 shows healing of a bone injury, after having applied a porous polyimide film to a bone injury site in a rat cranial bone loss model.
Fig. 5 shows healing of a bone injury, after having applied a porous polyimide film supporting bone marrow cells by single seeding and culturing, to a bone injury site in a rat cranial bone loss model.
Fig. 6 shows time-dependent change in new bone formation volume (BV) after application of a porous polyimide film supporting bone marrow cells by single or twice seeding and culturing, to a bone injury site in a rat cranial bone loss model.
Fig. 7 shows time-dependent change in bone mineral content (BMC) after application of a porous polyimide film supporting bone marrow cells by single or twice seeding and culturing, to a bone injury site in a rat cranial bone loss model.
Fig. 8 shows time-dependent change in bone mineral density (BMD) after application of a porous polyimide film supporting bone marrow cells by single or twice seeding and culturing, to a bone injury site in a rat cranial bone loss model.
Fig. 9 shows time-dependent changes in cell count after culturing of osteoblasts on TERUDERMIS (registered trademark), and a composite material of the porous polyimide film and TERUDERMIS (registered trademark).

DESCRIPTION OF EMBODIMENTS

One aspect of the present invention relates to an implant for treating a bone injury site, the implant comprising:

a porous polymer film; and
a layer containing a biocompatible material;

wherein the porous polymer film is a three-layer structure porous polymer film having a surface layer A and a surface layer B, the surface layers having a plurality of pores, and a macrovoid layer sandwiched between the surface layers A and B;

wherein an average pore diameter of the pores present in the surface layer A is smaller than an average pore diameter of the pores present in the surface layer B;

wherein the macrovoid layer has a partition wall bonded to the surface layers A and B, and a plurality of macrovoids surrounded by the partition wall and the surface layers A and B; and

wherein the pores in the surface layers A and B communicate with the macrovoids. This implant is also referred to as the "implant of the present invention" hereinbelow.

In the present specification, "implant" refers to an article or a device that is installed completely or partially within an animal's body by surgical treatment or a less invasive method, for example.

In the present specification, "bone injury" refers to a state of damage to bone tissue caused by trauma, fatigue, disease or the like, and it includes, for example, a state of damage only to the surface of bone tissue, as well as fracture and bone loss. In the present specification, "fracture" includes complete fracture where the bone has lost complete continuity, and incomplete fracture where the bone has not lost complete continuity. Aspects of fracture include closed fracture (simple fracture) in which the fracture site is not released out of the body, open fracture (complex fracture) in which the fracture site is released out of the body, single fracture in which one bone is disjoined at only one location, and compound fracture (double fracture) in which one bone is disjoined at multiple locations. The implant of the present invention may be applied to any of these aspects of fracture.

In the present specification, "healing of a bone injury site" means partial or complete improvement, repair or restoration of the damaged state of a site of bone injury. The term "healing of a fracture site" means partial or complete improvement, repair or restoration of a site where bone has been disjoined or lost. In addition, "accelerate healing of a bone injury" means to shorten the period for improvement, repair or restoration of the state of damage at a site where bone has been damaged, or to enlarge the area of improvement, repair or restoration of the damaged state.

The index for healing or acceleration of healing at a bone injury site may be adhesion of disjoined bone, decrease in the area or volume of bone loss, bone mineral density (BMD), bone mineral content (BMC), bone mass or new bone formation volume (BV).

An average pore diameter of the pore present on a surface layer A (hereinafter referred to as "surface A" or "mesh surface") in the porous polymer film used for the implant of the present invention is not particularly limited, but is, for example, 0.01 $\mu$m or more and less than 200 $\mu$m, 0.01 to 150 $\mu$m, 0.01 to 100 $\mu$m, 0.01 to 50 $\mu$m, 0.01 to 40 $\mu$m, 0.01 to 30 $\mu$m, 0.01 to 20 $\mu$m, or 0.01 to 15 $\mu$m, preferably 0.01 to 15 $\mu$m.

The average pore diameter of the pore present on a surface layer B (hereinafter referred to as "surface B" or "large pore surface") in the porous polymer film used for the implant of the present invention is not particularly limited so long as it is larger than the average pore diameter of the pore present on the surface A, but is, for example, greater than 5 $\mu$m and 200 $\mu$m or less, 20 $\mu$m to 100 $\mu$m, 30 $\mu$m to 100 $\mu$m, 40 $\mu$m to 100 $\mu$m, 50 $\mu$m to 100 $\mu$m, or 60 $\mu$m to 100 $\mu$m, preferably 20 $\mu$m to 100 $\mu$m.

The average pore diameter on the surface of the porous polymer film is determined by measuring pore area for 200 or more open pore portions, and calculated an average diameter according to the following Equation (1) from the average pore area assuming the pore shape as a perfect circle.

$$\text{Average Pore Diameter} = 2 \times \sqrt{(S a / \pi)} \qquad (1)$$

(wherein Sa represents the average value for the pore areas)

The thicknesses of the surface layers A and B are not particularly limited, but is, for example, 0.01 to 50 $\mu$m, preferably 0.01 to 20 $\mu$m.

The average pore diameter of macrovoids in the planar direction of the film in the macrovoid layer in the porous polymer film is not particularly limited but is, for example, 10 to 500 $\mu$m, preferably 10 to 100 $\mu$m, and more preferably 10 to 80 $\mu$m. The thicknesses of the partition wall in the macrovoid layer are not particularly limited, but is, for example, 0.01 to 50 $\mu$m, preferably 0.01 to 20 $\mu$m. In an embodiment, at least one partition wall in the macrovoid layer has one or two or more pores connecting the neighboring macrovoids and having the average pore diameter of 0.01 to 100 $\mu$m, preferably 0.01 to 50 $\mu$m. In another embodiment, the partition wall in the macrovoid layer has no pore.

The film thickness of the porous polymer film used for the implant of the present invention is not particularly limited, but may be 5 $\mu$m or more, 10 $\mu$m or more, 20 $\mu$m or more or 25 $\mu$m or more, and 500 $\mu$m or less, 300 $\mu$m or less, 100 $\mu$m or less, 75 $\mu$m or less, or 50 $\mu$m or less. It is preferably 5 to 500 $\mu$m, and more preferably 25 to 75 $\mu$m.

The film thickness of the porous polymer film used for the implant of the present invention can be measured using a contact thickness gauge.

The porosity of the porous polymer film used in the implant of the present invention is not particularly limited but is, for example, 40% or more and less than 95%.

The porosity of the porous polymer film used for the implant of the present invention can be determined by measuring the film thickness and mass of the porous film cut out to a prescribed size, and performing calculation from the basis weight according to the following Equation (2).

$$\text{Porosity (\%)} = (1 - w / (S \times d \times D)) \times 100 \qquad (2)$$

(wherein S represents the area of the porous film, d represents the film thickness, w represents the measured mass, and D represents the polymer density. The density is defined as 1.34 g/cm$^3$ when the polymer is a polyimide.)

The porous polymer film used for the present invention is preferably a porous polymer film which includes a three-layer structure porous polymer film having a surface layer A and a surface layer B, the surface layers having a plurality of pores, and a macrovoid layer sandwiched between the surface layers A and B; wherein the average pore diameter of the pore present on the surface layer A is 0.01 μm to 15 μm, and the average pore diameter of the pore present on the surface layer B is 20 μm to 100 μm; wherein the macrovoid layer has a partition wall bonded to the surface layers A and B, and a plurality of macrovoids surrounded by such a partition wall and the surface layers A and B, the thickness of the macrovoid layer, and the surface layers A and B is 0.01 to 20 μm; wherein the pores on the surface layers A and B communicate with the macrovoid, the total film thickness is 5 to 500 μm, and the porosity is 40% or more and less than 95%. In an embodiment, at least one partition wall in the macrovoide layer has one or two or more pores connecting the neighboring macrovoids with each other and having the average pore diameter of 0.01 to 100 μm, preferably 0.01 to 50 μm. In another embodiment, the partition wall does not have such pores.

The porous polymer film used for the implant of the present invention is preferably sterilized. The sterilization treatment is not particularly limited, but any sterilization treatment such as dry heat sterilization, steam sterilization, sterilization with a disinfectant such as ethanol, electromagnetic wave sterilization such as ultraviolet rays or gamma rays, and the like can be mentioned.

The porous polymer film used for the implant of the present invention is not particularly limited so long as it has the structural features described above and includes, preferably a porous polyimide film or porous polyethersulfone PES film (porous PES film).

Polyimide is a general term for polymers containing imide bonds in the repeating unit, and usually it refers to an aromatic polyimide in which aromatic compounds are directly linked by imide bonds. An aromatic polyimide has an aromatic-aromatic conjugated structure via an imide bond, and therefore has a strong rigid molecular structure, and since the imide bonds provide powerful intermolecular force, it has very high levels of thermal, mechanical and chemical properties.

In one embodiment, the porous polyimide film usable for the implant of the present invention is a porous polyimide film preferably containing polyimide (as a main component) obtained from tetracarboxylic dianhydride and diamine, more preferably a porous polyimide film composed of tetracarboxylic dianhydride and diamine. The phrase "including as the main component" means that it essentially contains no components other than the polyimide obtained from a tetracarboxylic dianhydride and a diamine, as constituent components of the porous polyimide film, or that it may contain them but they are additional components that do not affect the properties of the polyimide obtained from the tetracarboxylic dianhydride and diamine.

The porous polyimide film usable for the implant of the present invention includes a colored porous polyimide film obtained by forming a polyamic acid solution composition including a polyamic acid solution obtained from a tetracarboxylic acid component and a diamine component, and a coloring precursor, and then heat treating it at 250°C or higher.

A polyamic acid is obtained by polymerization of a tetracarboxylic acid component and a diamine component. A polyamic acid is a polyimide precursor that can be cyclized to a polyimide by thermal imidization or chemical imidization.

The polyamic acid used may be any one that does not have an effect on the invention, even if a portion of the amic acid is imidized. Specifically, the polyamic acid may be partially thermally imidized or chemically imidized.

When the polyamic acid is to be thermally imidized, there may be added to the polyamic acid solution, if necessary, an imidization catalyst, an organic phosphorus-containing compound, or fine particles such as inorganic fine particles or organic fine particles. In addition, when the polyamic acid is to be chemically imidized, there may be added to the polyamic acid solution, if necessary, a chemical imidization agent, a dehydrating agent, or fine particles such as inorganic fine particles or organic fine particles. Even if such components are added to the polyamic acid solution, they are preferably added under conditions that do not cause precipitation of the coloring precursor.

In this specification, a "coloring precursor" is a precursor that generates a colored substance by partial or total carbonization under heat treatment at 250°C or higher.

Coloring precursors usable for the production of the porous polyimide film are preferably uniformly dissolved or dispersed in a polyamic acid solution or polyimide solution and subjected to thermal decomposition by heat treatment at 250°C or higher, preferably 260°C or higher, even more preferably 280°C or higher and more preferably 300°C or higher, and preferably heat treatment in the presence of oxygen such as air, at 250°C, preferably 260°C or higher, even more preferably 280°C or higher and more preferably 300°C or higher, for carbonization to produce a colored substance, more preferably producing a black colored substance, with carbon-based coloring precursors being most preferred.

The coloring precursor, when being heated, first appears as a carbonized compound, but compositionally it contains other elements in addition to carbon, and also includes layered structures, aromatic crosslinked structures and tetrahedron carbon-containing disordered structures.

Carbon-based coloring precursors are not particularly restricted, and for example, they include tar or pitch such as petroleum tar, petroleum pitch, coal tar and coal pitch, coke, polymers obtained from acrylonitrile-containing monomers, ferrocene compounds (ferrocene and ferrocene derivatives), and the like. Of these, polymers obtained from acrylonitrile-containing monomers and/or ferrocene compounds are preferred, with polyacrylonitrile being preferred as a polymer obtained from an acrylonitrile-containing monomer.

Moreover, in another embodiment, examples of the porous polyimide film which may be used for the preset invention also include a porous polyimide film which can be obtained by molding a polyamic acid solution derived from a tetracarboxylic acid component and a diamine component followed by heat treatment without using the coloring precursor.

The porous polyimide film produced without using the coloring precursor may be produced, for example, by casting a polyamic acid solution into a film, the polyamic acid solution being composed of 3 to 60% by mass of polyamic acid having an intrinsic viscosity number of 1.0 to 3.0 and 40 to 97% by mass of an organic polar solvent, immersing or contacting in a coagulating solvent containing water as an essential component, and imidating the porous film of the polyamic acid by heat treatment. In this method, the coagulating solvent containing water as an essential component may be water, or a mixed solution containing 5% by mass or more and less than 100% by mass of water and more than 0% by mass and 95% by mass or less of an organic polar solvent. Further, after the imidation, one surface of the resulting porous polyimide film may be subjected to plasma treatment.

The tetracarboxylic dianhydride which may be used for the production of the porous polyimide film may be any tetracarboxylic dianhydride, selected as appropriate according to the properties desired. Specific examples of tetracarboxylic dianhydrides include biphenyltetracarboxylic dianhydrides such as pyromellitic dianhydride, 3,3',4,4'-biphenyltetracarboxylic dianhydride (s-BPDA) and 2,3,3',4'-biphenyltetracarboxylic dianhydride (a-BPDA), oxydiphthalic dianhydride, diphenylsulfone-3,4,3',4'-tetracarboxylic dianhydride, bis(3,4-dicarboxyphenyl)sulfide dianhydride, 2,2-bis(3,4-dicarboxyphenyl)-1,1,1,3,3,3-hexafluoropropane dianhydride, 2,3,3',4'-benzophenonetetracarboxylic dianhydride, 3,3',4,4'-benzophenonetetracarboxylic dianhydride, bis(3,4-dicarboxyphenyl)methane dianhydride, 2,2-bis(3,4-dicarboxyphenyl)propane dianhydride, p-phenylenebis(trimellitic acid monoester acid anhydride), p-biphenylenebis(trimellitic acid monoester acid anhydride), m-terphenyl-3,4,3',4'-tetracarboxylic dianhydride, p-terphenyl-3,4,3',4'-tetracarboxylic dianhydride, 1,3-bis(3,4-dicarboxyphenoxy)benzene dianhydride, 1,4-bis(3,4-dicarboxyphenoxy)benzene dianhydride, 1,4-bis(3,4-dicarboxyphenoxy)biphenyl dianhydride, 2,2-bis[(3,4-dicarboxyphenoxy)phenyl]propane dianhydride, 2,3,6,7-naphthalenetetracarboxylic dianhydride, 1,4,5,8-naphthalenetetracarboxylic dianhydride, 4,4'-(2,2-hexafluoroisopropylidene)diphthalic dianhydride, and the like. Also preferably used is an aromatic tetracarboxylic acid such as 2,3,3',4'-diphenylsulfonetetracarboxylic acid. These may be used alone or in appropriate combinations of two or more.

Particularly preferred among these are at least one type of aromatic tetracarboxylic dianhydride selected from the group consisting of biphenyltetracarboxylic dianhydride and pyromellitic dianhydride. As a biphenyltetracarboxylic dianhydride there may be suitably used 3,3',4,4'-biphenyltetracarboxylic dianhydride.

As diamine which may be used for the production of the porous polyimide film, any diamine may be used. Specific examples of diamines include the following.

1) Benzenediamines with one benzene nucleus, such as 1,4-diaminobenzene(paraphenylenediamine), 1,3-diaminobenzene, 2,4-diaminotoluene and 2,6-diaminotoluene;

2) diamines with two benzene nuclei, including diaminodiphenyl ethers such as 4,4'-diaminodiphenyl ether and 3,4'-diaminodiphenyl ether, and 4,4'-diaminodiphenylmethane, 3,3'-dimethyl-4,4'-diaminobiphenyl, 2,2'-dimethyl-4,4'-diaminobiphenyl, 2,2'-bis(trifluoromethyl)-4,4'-diaminobiphenyl, 3,3'-dimethyl-4,4'-diaminodiphenylmethane, 3,3'-dicarboxy-4,4'-diaminodiphenylmethane, 3,3',5,5'-tetramethyl-4,4'-diaminodiphenylmethane, bis(4-aminophenyl)sulfide, 4,4'-diaminobenzanilide, 3,3'-dichlorobenzidine, 3,3'-dimethylbenzidine, 2,2'-dimethylbenzidine, 3,3'-dimethoxybenzidine, 2,2'-dimethoxybenzidine, 3,3'-diaminodiphenyl ether, 3,4'-diaminodiphenyl ether, 4,4'-diaminodiphenyl ether, 3,3'-diaminodiphenyl sulfide, 3,4'-diaminodiphenyl sulfide, 4,4'-diaminodiphenyl sulfide, 3,3'-diaminodiphenylsulfone, 3,4'-diaminodiphenylsulfone, 4,4'-diaminodiphenylsulfone, 3,3'-diaminobenzophenone, 3,3'-diamino-4,4'-dichlorobenzophenone, 3,3'-diamino-4,4'-dimethoxybenzophenone, 3,3'-diaminodiphenylmethane, 3,4'-diaminodiphenylmethane, 4,4'-diaminodiphenylmethane, 2,2-bis(3-aminophenyl)propane, 2,2-bis(4-aminophenyl)propane, 2,2-bis(3-aminophenyl)-1,1,1,3,3,3-hexafluoropropane, 2,2-bis(4-aminophenyl)-1,1,1,3,3,3-hexafluoropropane, 3,3'-diaminodiphenyl sulfoxide, 3,4'-diaminodiphenyl sulfoxide and 4,4'-diaminodiphenyl sulfoxide;

3) diamines with three benzene nuclei, including 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene, 1,4-bis(3-aminophenoxy)benzene, 1,4-bis(4-aminophenoxy)benzene, 1,3-bis(3-aminophenoxy)-4-trifluoromethylbenzene, 3,3'-diamino-4-(4-phenyl)phenoxybenzophenone, 3,3'-diamino-4,4'-di(4-phenylphenoxy)benzophenone, 1,3-bis(3-aminophenyl sulfide)benzene, 1,3-bis(4-aminophenyl sulfide)benzene, 1,4-bis(4-aminophenyl sulfide)benzene, 1,3-bis(3-aminophenylsulfone)benzene, 1,3-bis(4-aminophenylsulfone)benzene, 1,4-bis(4-aminophenylsulfone)benzene, 1,3-bis[2-(4-aminophenyl)isopropyl]benzene, 1,4-bis[2-(3-aminophenyl)isopropyl]benzene and 1,4-bis[2-(4-aminophenyl)isopropyl]benzene;

4) diamines with four benzene nuclei, including 3,3'-bis(3-aminophenoxy)biphenyl, 3,3'-bis(4-aminophenoxy)biphenyl, 4,4'-bis(3-aminophenoxy)biphenyl, 4,4'-bis(4-aminophenoxy)biphenyl, bis[3-(3-aminophenoxy)phenyl]ether, bis[3-(4-aminophenoxy)phenyl]ether, bis[4-(3-aminophenoxy)phenyl]ether, bis[4-(4-aminophenoxy)phenyl]ether, bis[3-(3-aminophenoxy)phenyl]ketone, bis[3-(4-aminophenoxy)phenyl]ketone, bis[4-(3-aminophenoxy)phenyl]ketone, bis[4-(4-aminophenoxy)phenyl]ketone, bis[3-(3-aminophenoxy)phenyl] sulfide, bis[3-(4-aminophenoxy)phenyl] sulfide, bis[4-(3-aminophenoxy)phenyl] sulfide, bis[4-(4-aminophenoxy)phenyl] sulfide, bis[3-(3-aminophenoxy)phenyl]sulfone, bis[3-(4-aminophenoxy)phenyl]sulfone, bis[4-(3-aminophenoxy)phenyl]sulfone, bis[4-(4-aminophenoxy)phenyl]sulfone, bis[3-(3-aminophenoxy)phenyl]methane, bis[3-(4-aminophenoxy)phenyl]methane, bis[4-(3-aminophenoxy)phenyl]methane, bis[4-(4-aminophenoxy)phenyl]methane, 2,2-bis[3-(3-aminophenoxy)phenyl]propane, 2,2-bis[3-(4-aminophenoxy)phenyl]propane, 2,2-bis[4-(3-aminophenoxy)phenyl]propane, 2,2-bis[4-(4-aminophenoxy)phenyl]propane, 2,2-bis[3-(3-aminophenoxy)phenyl]-1,1,1,3,3,3-hexafluoropropane, 2,2-bis[3-(4-aminophenoxy)phenyl]-1,1,1,3,3,3-hexafluoropropane, 2,2-bis[4-(3-aminophenoxy)phenyl]-1,1,1,3,3,3-hexafluoropropane and 2,2-bis[4-(4-aminophenoxy)phenyl]-1,1,1,3,3,3-hexafluoropropane.

These may be used alone or in mixtures of two or more. The diamine used may be appropriately selected according to the properties desired.

Preferred among these are aromatic diamine compounds, with 3,3'-diaminodiphenyl ether, 3,4'-diaminodiphenyl ether, 4,4'-diaminodiphenyl ether, paraphenylenediamine, 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene and 1,4-bis(3-aminophenoxy)benzene being preferred for use. Particularly preferred is at least one type of diamine selected from the group consisting of benzenediamines, diaminodiphenyl ethers and bis(aminophenoxy)phenyl.

From the viewpoint of heat resistance and dimensional stability under high temperature, the porous polyimide film which may be used for the invention is preferably formed from a polyimide obtained by combination of a tetracarboxylic dianhydride and a diamine, having a glass transition temperature of 240°C or higher, or without a distinct transition point at 300°C or higher.

From the viewpoint of heat resistance and dimensional stability under high temperature, the porous polyimide film which may be used for the invention is preferably a porous polyimide film comprising one of the following aromatic polyimides:

(i) An aromatic polyimide comprising at least one tetracarboxylic acid unit selected from the group consisting of biphenyltetracarboxylic acid units and pyromellitic acid units, and an aromatic diamine unit,

(ii) an aromatic polyimide comprising a tetracarboxylic acid unit and at least one type of aromatic diamine unit selected from the group consisting of benzenediamine units, diaminodiphenyl ether units and bis(aminophenoxy)phenyl units,

and/or,

(iii) an aromatic polyimide comprising at least one type of tetracarboxylic acid unit selected from the group consisting of biphenyltetracarboxylic acid units and pyromellitic acid units, and at least one type of aromatic diamine unit selected from the group consisting of benzenediamine units, diaminodiphenyl ether units and bis(aminophenoxy)phenyl units.

The porous polyimide film used in the implant of the present invention is preferably a three-layer structure porous polyimide film having a surface layer A and a surface layer B, the surface layers having a plurality of pores, and a macrovoid layer sandwiched between the surface layers A and B; wherein an average pore diameter of the pores present in the surface layer A is 0.01 $\mu$m to 15 $\mu$m, and the mean pore diameter present on the surface layer B is 20 $\mu$m to 100 $\mu$m; wherein the macrovoid layer has a partition wall bonded to the surface layers A and B, and a plurality of macrovoids surrounded by such a partition wall and the surface layers A and B; wherein the thickness of the macrovoid layer, and the surface layers A and B is 0.01 to 20 $\mu$m, wherein the pores on the surface layers A and B communicate with the macrovoid, the total film thickness is 5 to 500 $\mu$m, and the porosity is 40% or more and less than 95%. In this case, at least one partition wall in the macrovoid layer has one or two or more pores connecting the neighboring macrovoids and having the average pore diameter of 0.01 to 100 $\mu$m, preferably 0.01 to 50 $\mu$m.

For example, porous polyimide films described in WO2010/038873, Japanese Unexamined Patent Publication (Kokai) No. 2011-219585 or Japanese Unexamined Patent Publication (Kokai) No. 2011-219586 may be used for the implant of the present invention.

The porous PES film which may be used for the present invention contains polyethersulfone and typically consists substantially of polyethersulfone. Polyethersulfone may be synthesized by the method known to those skilled in the art. For example, it may be produced by a method wherein a dihydric phenol, an alkaline metal compound and a dihalogenodiphenyl compound are subjected to polycondensation reaction in an organic polar solvent, a method wherein an alkaline metal di-salt of a dihydric phenol previously synthesized is subjected to polycondensation reaction dihalogenodiphenyl compound in an organic polar solvent or the like.

Examples of an alkaline metal compound include alkaline metal carbonate, alkaline metal hydroxide, alkaline metal hydride, alkaline metal alkoxide and the like. Particularly, sodium carbonate and potassium carbonate are preferred.

Examples of a dihydric phenol compound include hydroquinone, catechol, resorcin, 4,4'-biphenol, bis (hydroxyphenyl)alkanes (such as 2,2-bis(hydroxyphenyl)propane, and 2,2-bis(hydroxyphenyl)methane), dihydroxydiphenylsulfones, dihydroxydiphenyl ethers, or those mentioned above having at least one hydrogen on the benzene rings thereof substituted with a lower alkyl group such as a methyl group, an ethyl group, or a propyl group, or with a lower alkoxy group such as a methoxy group, or an ethoxy group. As the dihydric phenol compound, two or more of the aforementioned compounds may be mixed and used.

Polyethersulfone may be a commercially available product. Examples of a commercially available product include SUMIKAEXCEL 7600P, SUMIKAEXCEL 5900P (both manufactured by Sumitomo Chemical Company, Limited).

The logarithmic viscosity of the polyethersulfone is preferably 0.5 or more, more preferably 0.55 or more from the viewpoint of favorable formation of a macrovoid of the porous polyethersulfone film; and it is preferably 1.0 or less, more preferably 0.9 or less, further preferably 0.8 or less, particularly preferably 0.75 or less from the viewpoint of the easy production of a porous polyethersulfone film.

Further, from the viewpoints of heat resistance and dimensional stability under high temperature, it is preferred that the porous PES film or polyethersulfone as a raw material thereof has a glass transition temperature of 200°C or higher, or that a distinct glass transition temperature is not observed.

The method for producing the porous PES film which may be used for the present invention is not particularly limited. For example, the film may be produced by a method including the following steps:

a step in which polyethersulfone solution containing 0.3 to 60% by mass of polyethersulfone having logarithmic viscosity of 0.5 to 1.0 and 40 to 99.7% by mass of an organic polar solvent is casted into a film, immersed in or contacted with a coagulating solvent containing a poor solvent or non-solvent of polyethersulfone to produce a coagulated film having pores; and
a step in which the coagulated film having pores obtained in the above-mentioned step is heat-treated for coarsening of the aforementioned pores to obtain a porous PES film;
wherein the heat treatment includes the temperature of the coagulated film having the pores is raised higher than the glass transition temperature of the polyethersulfone, or up to 240°C or higher.

The porous PES film which can be used in the present invention is preferably a porous PES film having a surface layer A, a surface layer B, and a macrovoid layer sandwiched between the surface layers A and B,

wherein the macrovoid layer has a partition wall bonded to the surface layers A and B, and a plurality of macrovoids surrounded by such a partition wall and the surface layers A and B, the macrovoids having the average pore diameter in the planar direction of the film of 10 to 500 $\mu$m;

wherein the thickness of the macrovoid layer is 0.1 to 50 $\mu$m,

each of the surface layers A and B has a thickness of 0.1 to 50 $\mu$m,

wherein one of the surface layers A and B has a plurality of pores having the average pore diameter of more than 5 $\mu$m and 200 $\mu$m or less, while the other has a plurality of pores having the average pore diameter of 0.01 $\mu$m or more and less than 200 $\mu$m,

wherein one of the surface layers A and B has a surface aperture ratio of 15% or more while other has a surface aperture ratio of 10% or more,

wherein the pores of the surface layers A and B communicate with the macrovoids,

wherein the porous PES film has total film thickness of 5 to 500 $\mu$m and a porosity of 50 to 95%.

A "Layer containing a biocompatible material(s)" to be used in the implant of the present invention may be a layer comprising a biocompatible material, or a layer substantially comprising a biocompatible material. Note that in the case of the layer comprising a biocompatible material, the layer may contain the biocompatible material and an impurity to be contaminated with the layer upon preparation thereof, for example. Moreover, the "layer containing a biocompatible material" to be used in the implant of the present invention may be a layer comprising one biocompatible material (or a layer substantially comprising a biocompatible material), or a laminated product formed through lamination of layers comprising multiple biocompatible materials (or layers substantially comprising biocompatible materials). The thickness of the "layer containing a biocompatible material" is not particularly limited, and ranges from 10 $\mu$m to 5 cm and preferably ranges from 50 $\mu$m to 1 cm, for example.

Examples of a biocompatible material to be used in the implant of the present invention include, but are not limited to, collagen, silicone, fibronectin, laminin, polylysine, polylactide, polyglycolide, polycaprolactone, polyhydroxybutyrate, polylactide-co-caprolactone, polycarbonate, biodegradable polyurethane, polyetherester, polyesteramide, hydroxyapatite, a complex of collagen and $\beta$-TCP ($\beta$-tricalcium phosphate), and a combination thereof. The biocompatible material is preferably collagen. Examples of a layer containing collagen, which may be used herein include, but are not particularly limited to TERUDERMIS (registered trademark) and TERUPLUG (registered trademark) (both manufactured by Olympus Biomaterials Corp.).

In the implant of the present invention, the layer containing a biocompatible material may be arranged on surface layer A or surface layer B of the porous polymer film. Preferably the layer containing a biocompatible material is arranged on surface layer A of the porous polymer film. As a method for arranging the layer containing a biocompatible material on the surface layer of the porous polymer film, a method known by persons skilled in the art can be employed as appropriate. For example, this may be performed by coating with a solution containing a biocompatible material followed by drying. Moreover, this may also be performed by fixing the biocompatible material-containing layer formed in advance by thermocompression on the surface(s) of the porous polymer film. Furthermore, the surface(s) of the porous polymer film are heated, and then the biocompatible material-containing layer formed in advance may be fused.

Before arrangement of the layer containing a biocompatible material on a porous polymer film, the surfaces of the porous polymer film may be partially or entirely treated by a step of changing its physical properties. Examples of the step of changing physical properties include, but are not particularly limited to, alkaline treatment and calcium treatment.

An example of the step of treating the surfaces of a porous polymer film by alkaline treatment is, but is not limited to, a step of applying an alkaline substance such as sodium hydroxide or potassium hydroxide to the surfaces of a porous polymer film, and then changing the physical properties of the surfaces of the porous polymer film.

An example of the step of treating the surfaces of a porous polymer film by calcium treatment is, but is not limited to, a step of applying a substance containing calcium such as calcium chloride, calcium phosphate, or calcium fluoride to the surfaces of a porous polymer film, so as to change the physical properties of the surface of the porous polymer film.

Subsequent to the step of treating the surfaces of a porous polymer film by alkaline treatment, the step of treating the surfaces of a porous polyimide film by calcium treatment may be performed.

Cells may be supported in advance on the implant of the present invention. A porous polymer film to be used in the implant of the present invention has an advantage whereby cells can be retained and grown in its unique three dimensional structure. Cells of interest are supported on the implant of the present invention and then the implant is applied to a bone injury site, making it possible to accelerate treatment of the bone injury site.

Any method can be employed as a method by which cells are supported on the implant of the present invention, and the following methods can be employed, for example:

(A) an aspect comprising a step of seeding cells on a surface, on which a layer containing a biocompatible material is arranged, in the implant,

(B) an aspect comprising steps of
placing a cell suspension on a surface, on which a layer containing a biocompatible material is arranged, in the implant,
leaving the layer or moving the implant so as to accelerate the outflow of the fluid, or stimulating a part of the surface for the cell suspension to be absorbed by the porous polymer film of the implant, and,
retaining the cells in the cell suspension within the porous polymer film and causing the outflow of the water content, as well as,

(C) an aspect comprising steps of:

wetting one surface or both surfaces of the implant with a cell culture solution or a sterilized fluid;
loading the wetted implant with the cell suspension; and
retaining cells in the cell suspension within the implant and causing the outflow of the water content, and

(D) an aspect comprising a step of embedding the implant in a wound area or a loss section, and then directly applying a bone marrow aspirate thereto.

Cells seeded on the surface of the implant of the present invention enter the pores of the porous polymer film. Cells seeded on the surface of the implant of the present invention can stably grow and proliferate on the surface and/or the interior of the implant. Cells can take various different forms depending on the positions of films where cells grow and proliferate.

In the implant of the present invention, cells may be applied to the side of surface A or surface B of the porous polymer film. Preferably, cells are applied to surface A. More preferably, in the implant of the present invention, cells are applied to the surface on which the biocompatible material-containing layer of the implant of the present invention is arranged.

After cells are seeded on the surface of the implant of the present invention, the cells may be cultured in the implant according to purposes. The cultured cells are supported on the implant of the present invention.

The type of cells to be supported on the implant of the present invention is not particularly limited and any cells can be used herein. In general, cells derived from animals belonging to Mammalia referred to as mammals are preferably used. Examples of mammals include, but are not particularly limited to, preferably mice, rats, humans, monkeys, pigs, dogs, sheep, and goats.

The type of animal cells to be supported on the implant of the present invention is not limited to, but is preferably selected from the group consisting of pluripotent stem cells, tissue stem cells, somatic cells, and a combination thereof.

In the present specification, "pluripotent stem cells" refer to the generic name of stem cells having capability (multipotency) of differentiating into cells of all tissues. Examples of pluripotent stem cells include, but are not limited to, embryonic stem cells (ES cells), induced pluripotent stem cells (iPS cells), embryonic germ stem cells (EG cells), and germ stem cells (GS cells). Pluripotent stem cells are preferably ES cells or iPS cells, and are particularly preferably iPS cells since the iPS cells pose no ethical problem, for example. As pluripotent stem cells, any known pluripotent stem cells can be used and pluripotent stem cells described in WO2009/123349 (PCT/JP2009/057041) can be used, for example.

In the present specification, "tissue stem cells" refer to stem cells having capability (multipotency) of differentiating into various cell species, although cell lineages into which the cells can differentiate are limited to specific tissues. For example, hematopoietic stem cells in the bone marrow serve as the origin for blood cells, and neural stem cells differentiate into nerve cells. In addition to these examples, there are various types including hepatic stem cells for the formation of the liver, and skin stem cells for the formation of skin tissues. Preferably, tissue stem cells are selected from mesenchymal stem cells, hepatic stem cells, pancreatic stem cells, neural stem cells, skin stem cells, or hematopoietic stem cells.

In the present specification, "somatic cells" refer to cells other than germ cells from among cells composing a multicellular organism, and are not inherited via sexual reproduction to the next generation. Preferably, somatic cells are selected from hepatocytes, pancreatic cells, muscle cells, bone cells, osteoblasts, osteoclasts, chondrocytes, fat cells, skin cells, fibroblasts, pancreatic cells, renal cells, and pneumocytes, or, lymphocytes, erythrocytes, leukocytes, monocytes, and blood cells of macrophage or megakaryocyte.

In the present specification, "germ cells" refer to cells having a role of conveying genetic information to the next generation through reproduction. Examples thereof include gamete, specifically ova, egg cells, sperm, and sperm cells for sexual reproduction and spores for asexual reproduction.

In the present specification, cells may be selected from the group consisting of sarcoma cells, established cell line and transformed cells. "Sarcoma" is a cancer that arises from cells of connective tissue derived from nonepithelial cells such as bone, cartilage, fat, muscle, and blood, and examples thereof include soft tissue sarcoma and malignant bone neoplasm. Sarcoma cells are derived from sarcoma. "Established cell line" refers to cultured cells that are maintained *ex vivo* for long periods of time, so that the cells have certain stable properties, and semipermanent subculture thereof becomes possible. There are cell lines derived from various tissues of various biological species including human, such as PC12 cells (derived from rat adrenal medulla), CHO cells (derived from Chinese hamster ovary), HEK293 cells (derived from human embryonic kidney), HL-60 cells (derived from human leukocytes), HeLa cells (derived from human cervical cancer), Vero cells (derived from African green monkey kidney epithelial cells), MDCK cells (derived from canine renal tubular epithelial cells), and HepG2 cells (derived from human liver cancer). "Transformed cells" refer to cells with genetic properties modified by introducing nucleic acids (e.g., DNA) from outside the cells. Appropriate methods for transformation of animal cells, plant cells, and bacteria are each known.

Examples of cells to be supported on the implant of the present invention include preferably bone marrow cells, mesenchymal stem cells, osteoblasts and fibroblasts.

When bone marrow cells are supported on the implant of the present invention, bone marrow cells may be cells collected from mammalian bone marrow. Bone marrow cells can be collected from the bone marrow by known methods such as bone marrow aspiration and bone marrow flashing. Furthermore, bone marrow cells may be primary cultured cells of cells collected from mammalian bone marrow.

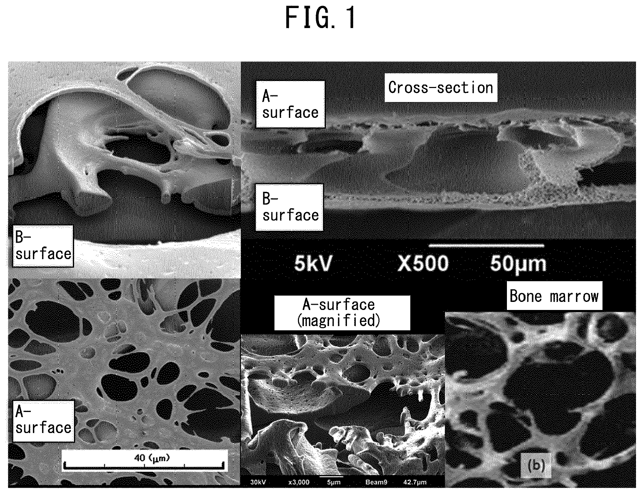

A porous polymer film to be used in the implant of the present invention has its unique three dimensional structure, the form of which resembles a bone marrow structure (for example, see Fig. 1). The present inventors have discovered that CD45 positive cells can be proliferated by seeding and culturing bone marrow cells on the porous polymer film, and that a cell mass having a property of differentiation analogous to that of the bone marrow is generated according to the three dimensional structure of the porous polymer film. The implant of the present invention may be prepared by seeding and culturing bone marrow cells thereon. Known medium may be used as appropriate for culturing.

The implant of the present invention may be an implant on which bone marrow cells collected from a subject with a bone injury to be treated are supported. With the use of such an implant for treatment of bone injuries, not only structural complementing of a bone injury site, but also recovery of bone functions including hematopoiesis are also possible.

As a method by which cells are supported on the implant of the present invention, a method comprising the following steps can be employed, for example:

(1) a step of applying a first cell group to the implant and then culturing cells, and
(2) a step of applying a second cell group to the implant after culturing in step (1), and then culturing cells, so that cells are supported, wherein the cells of the second cell group are bone marrow cells.

In the above method by which cells are supported, cells of the first cell group may be any type, for example, such cells may be cells selected from the group consisting of animal cells, insect cells, plant cells, yeast and bacteria. Animal cells are broadly divided into cells derived from animals belonging to the phylum vertebrata and cells derived from invertebrate (animals other than animals belonging to the phylum vertabrata). Cells of the first cell group are preferably bone marrow cells.

In one embodiment, the implant of the present invention further comprises metal mesh. When the implant comprises metal mesh, in the implant of the present invention, a layer containing a biocompatible material, a porous polymer film and metal mesh are arranged in this order.

In the present specification, "metal mesh" refers to mesh that is composed of metal components, and has many through holes on a sheet surface, and is not particularly limited, as long as it meets such requirements. Through the use of metal mesh, the mechanical strength of the implant of the present invention can be improved, and stable fields for healing bone can be established. Furthermore, metal mesh helps retain the shape of the implant at its fixed position. Metal mesh may be fixed on a porous polymer film by any fixing measure (e.g., suture, staple, and biocompatible screw).

Metal mesh is not particularly limited, and may be composed of, for example, titanium, stainless steel, titanium coated steel, titanium nitride or a combination thereof.

The implant of the present invention is rich in flexibility, and has an excellent degree of freedom in terms of shape, so that the implant can be cut into an arbitrary shape, shaped, or processed according to the conditions of a bone-injury affected part and then used.

Any method may be employed depending on the purpose in order to apply the implant of the present invention to a bone-injury affected part. Preferably, the implant of the present invention is implanted *in vivo* so that it comes into contact with a bone-injury affected part. The implant of the present invention may contact with the whole bone-injury affected part, or a porous polyimide film may contact with a portion of the bone injury affected part. The implant of the present invention is preferably used by implanting the implant *in vivo* so that the surface on which a biocompatible material-containing layer is arranged contacts with the bone injury affected part.

The implant of the present invention can be effectively used for treatment of a large bone loss site or a complicated fracture site that has been difficult to be treated by conventional techniques. The implant of the present invention may be applied so as to cover the whole or a portion of a bone-injury affected part, and is not particularly required to be fixed using another material. However, the implant may be fixed to a living tissue depending on the purpose using fixing measures such as suture, staple, or biocompatible screw.

One aspect of the present invention relates to a kit for treating a bone injury, comprisig the implant of the present invention, materials required for bone injury treatment surgery, and instruction manuals. An embodiment is, but is not limited to, a package containing a single or multiple sheets of the sterilized implant of the present invention, which is/are stored in transparent pouches in modes directly usable for treatment of bone injuries, or a kit in an integrated mode of a film and fluid including a sealed pouch containing the implant of the present invention and the sterilized fluid, whereby efficient seeding of cells is possible. Examples of materials required for surgery for treatment of bone injuries include, but are not particularly limited to, fixing measures (e.g., suture, staple or biocompatible screw) for fixing the implant of the present invention to a bone-injury affected part.

One aspect of the present invention relates to a method for treating a bone injury site, the method comprising applying the implant of the present invention to the bone injury site.

The present invention will now be explained in greater detail by Examples. It is to be understood, however, that the invention is not limited to these Examples. A person skilled in the art may easily implement modifications and changes to the invention based on the description in the present specification, and these are also encompassed within the technical scope of the invention.

EXAMPLES

The porous polyimide films used in the following examples were prepared by forming a polyamic acid solution composition including a polyamic acid solution obtained from 3,3',4,4'-biphenyltetracarboxylic dianhydride (s-BPDA) as a tetracarboxylic acid component and 4,4'-diaminodiphenyl ether (ODA) as a diamine component, and polyacrylamide as a coloring precursor, and performing heat treatment at 250°C or higher. The resulting porous polyimide film was a three-layer structure porous polyimide film having a surface layer A and a surface layer B, the surface layers having a plurality of pores, and a macrovoid layer sandwiched between the surface layers A and B; wherein the average pore diameter of the pore present on the surface layer A was 6 $\mu$m, the average pore diameter of the pore present on the surface layer B was 46 $\mu$m, and the film thickness was 25 $\mu$m, and the porosity was 73%.

Example 1

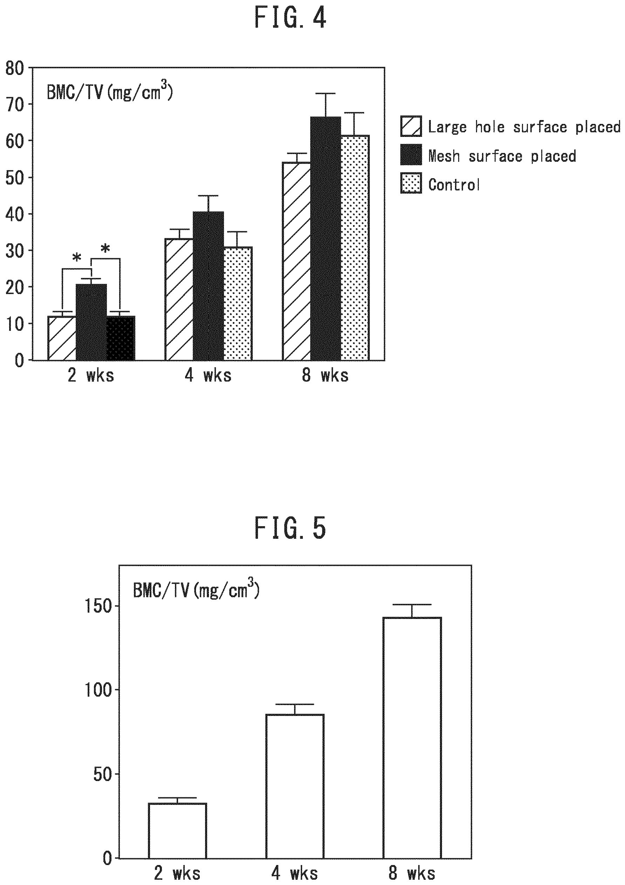

After general anesthesia of 9-week-old LEW rats with 2-3% isoflurane, they were subjected to infiltration anesthesia in the operating field, with 1/10,000 epinephrine-containing lidocaine. A region wider than the incision site was shaved, and then an incision was made at the top of the head on a straight line up to the subperiosteum, for sufficient delineation of the operating field. After detaching the dermal periosteal flap and exposing the parietal bone, a trephine bur was used to form two 4 mm-diameter circular bone loss sections under poured sterile physiological saline. After covering the loss section with a sterilized porous polyimide film, the dermal periosteal flap was reinstated and sutured with a nylon thread. The mesh surface (A-surface) of a porous polyimide film was placed in the wound area to prepare one model, and the large-hole surface (B-surface) was placed to prepare another model. After 2 weeks, 4 weeks and 8 weeks, the state of healing of the loss section was measured. The measured parameters were bone mineral density (BMD) (mg/cm$^3$), bone mineral content (BMC) (mg), new bone formation volume (BV) (cm3), set ROI volume (TV) (cm3), new bone formation percentage (BV/TV) (%) and clinical BMD (BMC/TV) (mg/cm$^3$). As regards early osteogenesis in the mesh surface-placed model, it was confirmed that the model in which the mesh surface had been placed in the wound area had significantly accelerated osteogenesis. The results are shown in Fig. 4. In Fig. 4, the control group is the group without covering of the bone loss section with a porous polyimide film.

Example 2

After harvesting femora and tibia of 8-week-old GFP transgenic rats and cutting off both ends of each bone, bone marrow cell masses were harvested by flushing with 10% FBS-added DMEM medium. The cell masses were pulverized by pipetting, and $1.0 \times 10^6$ bone marrow cells were seeded on the mesh surface (A-surface) of a 1.5 cm-square porous polyimide film and stationary cultured for 5 days in DMEM medium containing 10% FBS. On the 6th day, the cell-adhered porous polyimide film was rinsed with phosphate buffer and further cultured for 1 day, after only changing the medium to DMEM. After general anesthesia of 9-week-old nude rats with 2-3% isoflurane, they were subjected to infiltration anesthesia in the field of operation, with 1/10,000 epinephrine-containing lidocaine. A region wider than the incision site was shaved, and then an incision was made at the top of the head on a straight line up to the subperiosteum, for sufficient delineation of the operating field. After detaching the dermal periosteal flap and exposing the parietal bone, a trephine bur was used to form two 4 mm-diameter circular bone loss sections under poured sterile physiological saline. The loss section was covered by the porous polyimide film on which the cells had been adhered and then cultured, in a manner so as to place the mesh surface (A-surface) in the wound area. After 2 weeks, 4 weeks and 8 weeks, the condition of healing of the loss section was measured, and healing of the wound area was periodically confirmed. The results are shown in Fig. 5.

Example 3

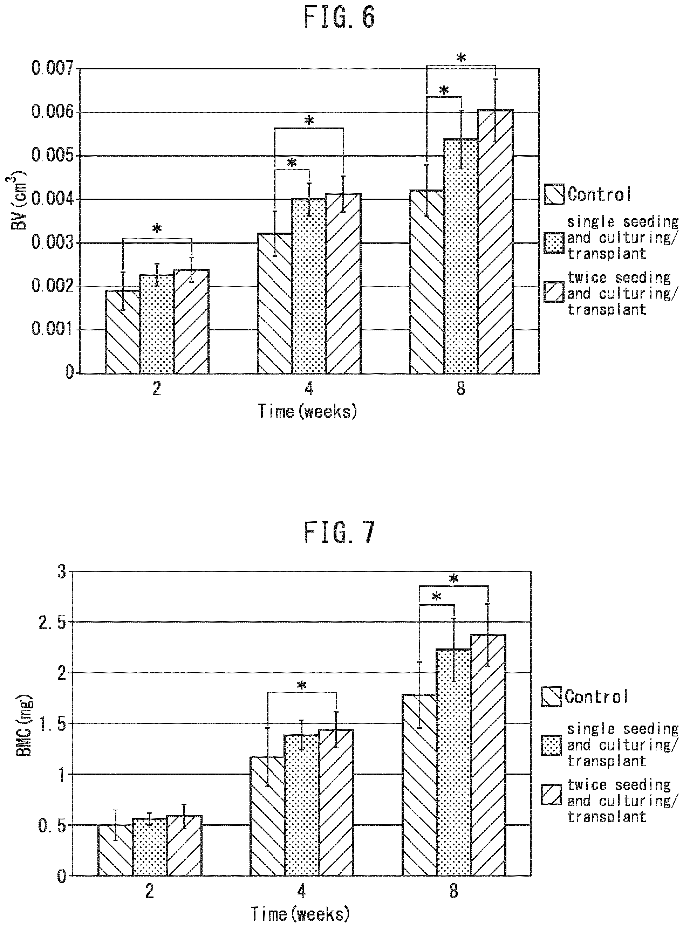

After harvesting femora and tibia of 8-week-old GFP transgenic rats and cutting off both ends of each bone, bone marrow cell masses were harvested by flushing with 10% FBS-added DMEM medium. The cell masses were pulverized by pipetting, and $1.0 \times 10^6$ bone marrow cells were seeded on the mesh surface (A-surface) of a 1.5 cm-square porous polyimide film and stationary cultured for 5 days in DMEM medium containing 10% FBS. After exchanging the DMEM medium, culturing was continued for 1 day. Next, $1.0 \times 10^6$ bone marrow cells were seeded on the mesh surface (A-surface) of a porous polyimide film and stationary cultured for 5 days in DMEM medium containing 10% FBS. On the 6th day, the cell-adhered porous polyimide film was rinsed with phosphate buffer and further cultured for 1 day, after only changing the medium to DMEM. After general anesthesia of 9-week-old nude rats with 2-3% isoflurane, they were subjected to infiltration anesthesia in the field of operation, with 1/10,000 epinephrine-containing lidocaine. A region wider than the incision site was shaved, and then an incision was made at the top of the head on a straight line up to the subperiosteum, for sufficient delineation of the operating field. After detaching the dermal periosteal flap and exposing the parietal bone, a trephine bur was used to form two 4 mm-diameter circular bone loss sections under poured sterile physiological saline. The loss section was covered by the porous polyimide film on which the cells had been adhered and then cultured, in a manner so as to place the mesh surface (A-surface) in the wound area. After 2 weeks, 4 weeks and 8 weeks, the condition of healing of the loss section was measured, and healing of the wound area was periodically confirmed. The results are shown in Figs. 6 to 8.

Example 4

Conjugation of porous polyimide film and TERUDERMIS (registered trademark)

TERUDERMIS (registered trademark) graft for lost dermis (ALCARE Co., Ltd.) was opened in a sterile manner and cut with scissors into the same size as that of a porous polyimide film to be tested. The porous polyimide film was placed on the upper surface (silicone layer side) of TERUDERMIS (registered trademark), and then fused to the silicone layer with tweezers heated with a flame.

Example 5

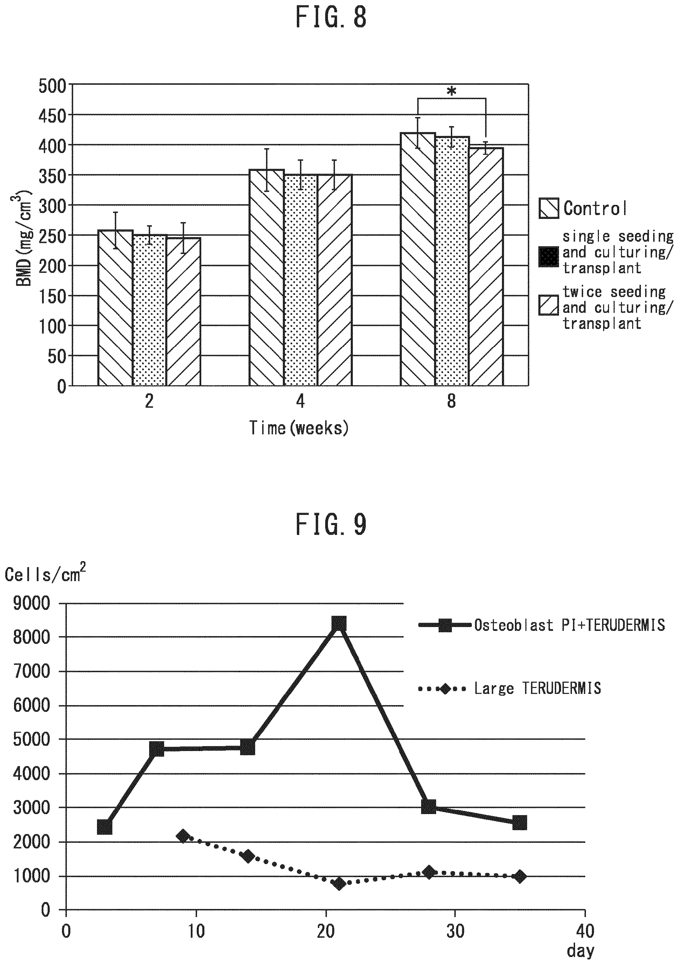

Time-dependent changes in cell count upon culturing of osteoblasts on TERUDERMIS (registered trademark), and on composite material of porous polyimide film and TERUDERMIS (registered trademark)
The composite material (sterilized, 1.4cm-square) of the porous polyimide film and TERUDERMIS (registered trademark) produced in Example 4 was placed in a 2 cm $\times$ 2 cm sterilized square container (Thermo Fisher Scientific, cat. 103). $4 \times 10^4$ cells were seeded, 4 ml of osteoblast growth medium was added, cells were continuously cultured within a $CO_2$ incubator while exchanging media twice a week. Furthermore, 2.5 cm $\times$ 2.5 cm TERUDERMIS (registered trademark) was placed in a $20cm^2$ petri dish, and then osteoblasts were seeded and cultured under similar conditions. On days 3, 7, 14, 21, 28, and 35 and in the case of TERUDERMIS, on days 9, 14, 21, 28, and 35 after seeding, cell count was determined using CCK8 and proliferation behavior was observed. Results are depicted in Fig. 9. During the culture period, almost no cell proliferation and no migration were observed on TERUDERMIS (registered trademark). However, increases in cell count were observed throughout the culture period by only coexistence with the porous polyimide film.

INDUSTRIAL APPLICABILITY

With the implant of the present invention, bone injuries including large loss sites and complicated shape sites can be conveniently and effectively treated.

**Claims**

1.   An implant for treating a bone injury site, the implant comprising:

   a porous polymer film; and
   a layer containing a biocompatible material;

   wherein the porous polymer film is a three-layer structure porous polymer film having a surface layer A and a surface layer B, the surface layers having a plurality of pores, and a macrovoid layer sandwiched between the surface layers A and B;
   wherein an average pore diameter of the pores present in the surface layer A is smaller than an average pore diameter of the pores present in the surface layer B;
   wherein the macrovoid layer has a partition wall bonded to the surface layers A and B, and a plurality of macrovoids surrounded by the partition wall and the surface layers A and B; and
   wherein the pores in the surface layers A and B communicate with the macrovoids.

2.   The implant according to claim 1, wherein the layer containing the biocompatible material is arranged on the surface layer A of the porous polymer film.

3.   The implant according to claim 1 or 2, wherein the biocompatible material is a compound selected from the group consisting of collagen, silicone, fibronectin, laminin, polylysine, polylactide, polyglycolide, polycaprolactone, polyhydroxybutyrate, polylactide-co-caprolactone, polycarbonate, biodegradable polyurethane, polyetherester, polyesteramide, hydroxyapatite, a complex of collagen and β-TCP, and a combination thereof.

4.   The implant according to any one of claims 1 to 3, wherein the biocompatible material is collagen.

5. The implant according to any one of claims 1 to 4, wherein an average pore diameter of the surface layer A is 0.01 to 50 $\mu$m.

6. The implant according to any one of claims 1 to 5, wherein an average pore diameter of the surface layer B is 20 to 100 $\mu$m.

7. The implant according to any one of claims 1 to 6, wherein a film thickness of the porous polymer film is 5 to 500 $\mu$m.

8. The implant according to any one of claims 1 to 7, wherein the porous polymer film is a porous polyimide film.

9. The implant according to claim 8, wherein the porous polyimide film is a porous polyimide film comprising a polyimide derived from tetracarboxylic dianhydride and diamine.

10. The implant according to claim 8 or 9, wherein the porous polyimide film is a colored porous polyimide film that is obtained by molding a polyamic acid solution composition comprising a polyamic acid solution derived from tetracarboxylic dianhydride and diamine, and a colored precursor, and subsequently heat-treating the resultant composition at 250°C or higher.

11. The implant according to any one of claims 1 to 7, wherein the porous polymer film is a porous polyethersulfone film.

12. The implant according to any one of claims 1 to 11, on which cells have been supported.

13. The implant according to claim 12, wherein the cells are bone marrow cells, mesenchymal stem cells, fibroblasts, osteoblasts, or cells derived therefrom.

14. The implant according to any one of claims 1 to 13, further comprising a metal mesh, wherein the layer containing the biocompatible material, the porous polymer film and the metal mesh are arranged in this order.

15. The implant according to claim 14, wherein the metal mesh comprises titanium, stainless steel, titanium-coated steel, titanium nitride or a combination thereof.

16. A kit for treating a bone injury, the kit comprising:

   the implant according to any one of claims 1 to 15;
   a material required for a bone injury treatment surgery; and
   an instruction manual.

17. A method for treating a bone injury site, the method comprising applying the implant according to any one of claims 1 to 15 to the bone injury site.

# FIG. 1

# FIG. 2

Loss site
formation

Loss site
covered

# FIG. 3

Loss site formation

Loss site covered

## FIG. 4

## FIG. 5

# FIG. 6

# FIG. 7

# FIG. 8

# FIG. 9

<table>
<tr><td colspan="2">**INTERNATIONAL SEARCH REPORT**</td><td>International application No.<br>PCT/JP2017/026905</td></tr>
</table>

**A. CLASSIFICATION OF SUBJECT MATTER**
*A61L27/18*(2006.01)i, *A61K35/28*(2015.01)i, *A61K35/32*(2015.01)i,
*A61K35/33*(2015.01)i, *A61L27/06*(2006.01)i, *A61L27/12*(2006.01)i,
*A61L27/22*(2006.01)i, *A61L27/24*(2006.01)i, *A61L27/38*(2006.01)i,
According to International Patent Classification (IPC) or to both national classification and IPC

**B. FIELDS SEARCHED**

Minimum documentation searched (classification system followed by classification symbols)
A61L27/18, A61K35/28, A61K35/32, A61K35/33, A61L27/06, A61L27/12,
A61L27/22, A61L27/24, A61L27/38, A61L27/44, A61L27/56

Documentation searched other than minimum documentation to the extent that such documents are included in the fields searched
Jitsuyo Shinan Koho          1922–1996   Jitsuyo Shinan Toroku Koho   1996–2017
Kokai Jitsuyo Shinan Koho    1971–2017   Toroku Jitsuyo Shinan Koho   1994–2017

Electronic data base consulted during the international search (name of data base and, where practicable, search terms used)
JSTPlus/JMEDPlus/JST7580(JDreamIII), CAplus/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
|---|---|---|
| P,X | WO 2016/121771 A1 (Ube Industries, Ltd.), 04 August 2016 (04.08.2016), claims 1 to 42; paragraphs [0020] to [0040], [0109] to [0113], [0137]; examples 1 to 5 (Family: none) | 1-10,12-17 |
| Y | WO 2015/012415 A1 (Ube Industries, Ltd.), 29 January 2015 (29.01.2015), claims 1 to 23; paragraphs [0070] to [0097]; examples 1 to 12 & US 2017/0037365 A1 claims 1 to 23; paragraphs [0112] to [0140]; examples 1 to 12 & EP 3026108 A1          & CN 105452440 A & KR 10-2016-0034303 A | 1-10,12-17 |

| ☒ Further documents are listed in the continuation of Box C. | ☐ See patent family annex. |

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>21 August 2017 (21.08.17) | Date of mailing of the international search report<br>29 August 2017 (29.08.17) |
|---|---|
| Name and mailing address of the ISA/<br>Japan Patent Office<br>3-4-3,Kasumigaseki,Chiyoda-ku,<br>Tokyo 100-8915,Japan | Authorized officer<br><br><br>Telephone No. |

Form PCT/ISA/210 (second sheet) (January 2015)

**INTERNATIONAL SEARCH REPORT**

| International application No. |
|---|
| PCT/JP2017/026905 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 63-196286 A  (Sumitomo Electric Industries, Ltd.), 15 August 1988 (15.08.1988), page 2, left column, line 11 to right column, 2nd paragraph; example 1 (Family: none) | 1-10,12-17 |
| Y | JP 63-198978 A  (Sumitomo Electric Industries, Ltd.), 17 August 1988 (17.08.1988), page 2, upper right column, lines 1 to 12; example 1 (Family: none) | 1-10,12-17 |
| Y | WO 2003/018077 A1  (National Institute of Advanced Industrial Science and Technology), 06 March 2003 (06.03.2003), claims 1 to 8; examples 1 to 5 & US 2004/241145 A1 claims 1 to 8; examples 1 to 5 & EP 1433487 A1 | 1-10,12-17 |
| Y | JP 2014-4327 A  (Yoshinori KUBOKI), 16 January 2014 (16.01.2014), claims 1 to 6 (Family: none) | 14-17 |
| A | ILMARINEN, T. et al., Ultrathin Polyimide Membrane as Cell Carrier for Subretinal Transplantation of Human Embryonic Stem Cell Derived Retinal Pigment Epithelium, PLOS ONE, 2015, Vol.10, No.11, e0143669, p.1-18, ISSN 1932-6203 | 1-10,12-17 |
| A | TAO, C.T. et al., Polyetherimide membrane formation by the cononsolvent system and its biocompatibility of MG63 cell line, Journal of Membrane Science, 2006, Vol.269, p.66-74, ISSN 0376-7388 | 1-10,12-17 |
| A | VAN VLIERBERGHE, S. et al., Surface Modification of Polyimide Sheets for Regenerative Medicine Applications, Biomacromolecules, 2010, Vol.11, p.2731-2739, ISSN 1525-7797 | 1-10,12-17 |
| A | NAGANO, M. et al, Bone bonding ability of an apatite-coated polymer produced using a biomimetic method: A mechanical and histological study in vivo, Journal of Biomedical Materials Research, 1996, Vol.31, p.487-494, Abstract, ISSN 0021-9304 | 11 |

Form PCT/ISA/210 (continuation of second sheet) (January 2015)

**INTERNATIONAL SEARCH REPORT**

International application No.

PCT/JP2017/026905

C (Continuation).  DOCUMENTS CONSIDERED TO BE RELEVANT

| Category* | Citation of document, with indication, where appropriate, of the relevant passages | Relevant to claim No. |
|---|---|---|
| P,Y | NOREIKAITE, A. et al., Scaffold design for artificail tissue with bone marrow stem cells, MEDICINA, 2017.07.13, [Epub ahead of print], ISSN 1010-660X | 1-10,12-17 |

Form PCT/ISA/210 (continuation of second sheet) (January 2015)

**INTERNATIONAL SEARCH REPORT**

International application No.

PCT/JP2017/026905

Continuation of A. CLASSIFICATION OF SUBJECT MATTER
  (International Patent Classification (IPC))

*A61L27/44*(2006.01)i, *A61L27/56*(2006.01)i

　　　　　(According to International Patent Classification (IPC) or to both national classification and IPC)

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3029266 B
- JP 4412537 B
- JP 4950269 B
- WO 2010038873 A
- JP 2011219585 A
- JP 2011219586 A
- WO 2015012415 A
- WO 2009123349 A
- JP 2009057041 W

**Non-patent literature cited in the description**

- **F.G. LYONS et al.** *Biomaterials,* 2010, vol. 31, 9232-9243
- **S. WU et al.** *Materials Science and Engineering,* 2014, vol. R 80, 1-36